# EUROPÄISCHE PATENTANMELDUNG

(11) **EP 4 614 020 A1**
(43) Veröffentlichungstag der Anmeldung: **10.09.2025**
(21) Anmeldenummer: 25158304.3
(22) Anmeldetag: 17.02.2025
(51) Int. Cl.: F16C 32/04, A61M 60/822, F04D 13/06, H02K 7/09, H02K 19/06, A61M 60/104, A61M 60/122, A61M 60/216, A61M 60/422

(54) **MAGNETLAGERVORRICHTUNG UND ELEKTROMAGNETISCHER DREHANTRIEB**

(30) Priorität: 05.03.2024 EP 24161376
(71) Anmelder: Levitronix GmbH, 8048 Zürich (CH)
(72) Erfinder: Steinert, Daniel, 8180 Bülach (CH)
(74) Vertreter: IPS Irsch AG

(57) **Zusammenfassung**

Es wird eine Magnetlagervorrichtung vorgeschlagen zur berührungslos magnetischen Lagerung eines Rotors (3), der einen scheibenförmigen oder ringförmigen magnetisch wirksamen Kern (31) umfasst, wobei die Magnetlagervorrichtung einen Stator (2) mit einer becherförmigen Ausnehmung (211) aufweist, die an einem axialen Ende des Stators (2) angeordnet ist, und in die der Rotor (3) einsetzbar ist, wobei der Stator (2) eine Mehrzahl von Spulenkernen (25) aufweist, von denen jeder einen Längsschenkel (26) und ein Polstück (27) aufweist, wobei sich jeder Längsschenkel (26) von einem ersten Ende (261) in einer axialen Richtung (A) bis zu einem zweiten Ende (262) erstreckt, wobei am zweiten Ende (262) eine Kontaktfläche (271) angeordnet ist, wobei sich jedes Polstück (27) von der Kontaktfläche (271) zumindest teilweise in einer radialer Richtung (R) bis zu einer Stirnfläche (272) erstreckt, wobei die radiale Richtung (R) senkrecht zur axialen Richtung (A) ist, wobei die Stirnfläche (272) um die becherförmige Ausnehmung (211) herum angeordnet sind, und wobei an jedem Längsschenkel (26) mindestens eine konzentrierte Wicklung (61) angeordnet ist, welche den jeweiligen Längsschenkel (26) umgibt, wobei die Längsschenkel (26) aus einem ersten Material gefertigt sind und die Polstücke (27) aus einem zweiten Material gefertigt sind, wobei das erste Material und das zweite Material unterschiedlich sind. Ferner wird ein elektromagnetischer Drehantrieb mit einer solchen Magnetlagervorrichtung vorgeschlagen.

## Beschreibung

Die Erfindung betrifft eine Magnetlagervorrichtung gemäss dem Oberbegriff des unabhängigen Patentanspruchs und einen elektromagnetischen Drehantrieb mit einer solchen Magnetlagervorrichtung.

Magnetlagervorrichtungen zur berührungslos magnetischen Lagerung eines Rotors haben den Vorteil, dass sie ohne mechanische Lager für den Rotor auskommen. Der Rotor wird mittels magnetischer Kräfte gelagert bzw. stabilisiert, die von einem Stator der Magnetlagervorrichtung generiert werden. Aufgrund der Abwesenheit von mechanischen Lagern eignen sich solche Magnetlagervorrichtungen insbesondere für Pump-, Misch-, Zentrifugier- oder Rührvorrichtungen, mit denen sehr empfindliche Substanzen gefördert werden, beispielsweise Blutpumpen, oder bei denen sehr hohe Anforderungen an die Reinheit gestellt werden, beispielsweise in der pharmazeutischen Industrie oder in der biotechnologischen Industrie, oder mit denen abrasive oder aggressive Substanzen gefördert werden, welche mechanische Lager sehr schnell zerstören würden, beispielsweise Pumpen oder Mischer für Slurry, Schwefel-, Phosphorsäure oder andere Chemikalien in der Halbleiterindustrie.

In der biotechnologischen Industrie werden solche Magnetlagervorrichtungen beispielsweise im Zusammenhang mit Bioreaktoren eingesetzt, z. B. bei Zentrifugalpumpen zum Fördern der Fluide in den oder aus dem Bioreaktor, oder bei Mischvorrichtungen, welche die Fluide in dem Bioreaktor durchmischen. In der Halbleiterindustrie werden solche Magnetlagervorrichtung nicht nur für das Fördern aggressiver oder abrasiver Substanzen verwendet, sondern beispielsweise auch für Rotationsvorrichtungen, mit denen Wafer rotiert werden.

Auch ist es bekannt, Magnetlagervorrichtungen für Viskosimeter zu verwenden.

Eine vorteilhafte und an sich bekannte Ausführungsform einer Magnetlagervorrichtung ist die Ausführung in Tempelbauweise, auf die sich auch die vorliegende Erfindung bezieht.

Das Charakteristische der Tempelbauweise ist es, dass der Stator der Magnetlagervorrichtung eine Mehrzahl von Spulenkernen aufweist, von denen jeder einen Längsschenkel umfasst, der sich von einem ersten Ende in einer axialen Richtung bis zu einem zweiten Ende erstreckt. Mit der axialen Richtung ist dabei diejenige Richtung gemeint, welche durch die Solldrehachse des Rotors definiert ist, welcher mit der Magnetlagervorrichtung gelagert wird. Die Solldrehachse ist diejenige Drehachse, um welche der Rotor im Betriebszustand rotiert, wenn er bezüglich des Stators in einer zentrierten und unverkippten Position ist. Jeder Spulenkern umfasst zusätzlich zu dem Längsschenkel einen Querschenkel, auch Polstück genannt, welcher jeweils an dem zweiten Ende des Längsschenkels angeordnet ist, und welcher sich in radialer Richtung - üblicherweise nach innen - erstreckt, wobei die radiale Richtung senkrecht zur axialen Richtung ist. Der Querschenkel erstreckt sich also im Wesentlichen rechtwinklig zum Längsschenkel. Die Spulenkerne haben jeweils die Form eines L, wobei die Querschenkel die kurzen Schenkel des L bilden. Der zu lagernde Rotor ist dann zwischen den Querschenkeln angeordnet.

Die Mehrzahl der Längsschenkel, die sich in axialer Richtung erstrecken und an die Säulen eines Tempels erinnern, hat dieser Bauweise ihren Namen gegeben.

In einer Ausführungsform hat der Stator der Magnetlagervorrichtung beispielsweise sechs Spulenkerne, die kreisförmig und äquidistant um eine becherförmige Ausnehmung herum angeordnet sind, in welche der Rotor eingesetzt werden kann. Die ersten Enden der Längsschenkel sind üblicherweise in Umfangsichtung durch einen Rückschluss verbunden, welcher der magnetischen Flussführung dient. Der zu lagernde Rotor umfasst einen magnetisch wirksamen Kern, beispielsweise eine permanentmagnetische Scheibe oder einen permanentmagnetischen Ring, der zwischen den radial innenliegenden Enden der Querschenkel angeordnet ist und im Betriebszustand um die axiale Richtung rotiert, wobei der Rotor berührungslos magnetisch bezüglich des Stators gelagert ist.

Für solche Magnetlagervorrichtungen ist es nicht notwendiger Weise so, dass der magnetisch wirksame Kern des Rotors permanentmagnetisch ausgestaltet sein muss. Es sind auch solche Ausgestaltungen bekannt, bei denen der magnetisch wirksame Kern des Rotors permanentmagnetfrei, also ohne Permanentmagnete ausgestaltet ist. Der magnetisch wirksame Kern des Rotors ist dann beispielsweise ferromagnetisch ausgestaltet und besteht beispielsweise aus Eisen, Nickel-Eisen, Kobalt-Eisen, Silizium-Eisen, Mu-Metall oder einem anderen ferromagnetischen Werkstoff.

Ferner sind Ausgestaltungen möglich, bei denen der magnetisch wirksame Kern des Rotors sowohl ferromagnetische Materialien als auch permanentmagnetische Materialien umfasst. Beispielsweise können Permanentmagnete in einen ferromagnetischen Grundkörper eingelegt bzw. eingesetzt werden. Solche Ausgestaltungen sind z.B. vorteilhaft, wenn man bei grossen Rotoren die Kosten durch Einsparen von permanentmagnetischem Material reduzieren will.

Um die für die berührungslos magnetische Lagerung des Rotors notwendigen elektromagnetischen Felder zu erzeugen, tragen die Längsschenkel Wicklungen. Die Wicklungen sind beispielsweise so ausgestaltet, dass um jeden Längsschenkel herum eine konzentrierte Wicklung gewickelt ist, das heisst, die Spulenachse jeder konzentrierten Wicklung erstreckt sich jeweils in axialer Richtung. Dabei ist es typisch für die Tempelbauweise, dass die Spulenachsen der konzentrierten Wicklungen in der axialen Richtung verlaufen und, dass die konzentrierten Wicklungen nicht in der radialen Ebene angeordnet sind, in welcher der Rotor bzw. der magnetisch wirksame Kern des Rotors im Betriebszustand gelagert wird.

Es sind Ausgestaltungen möglich, bei denen auf jedem Längsschenkel genau eine konzentrierte Wicklung angeordnet ist. In anderen Ausgestaltungen sind auf jedem Längsschenkel mehrere, beispielsweise genau zwei konzentrierte Wicklungen vorgesehen. Auch sind Ausgestaltungen möglich, bei denen Wicklungen vorgesehen sind, die um zwei in Umfangsrichtung benachbarte Längsschenkel herumgewickelt sind, sodass sich diese beiden benachbarten Längsschenkel beide im Innenraum der konzentrierten Wicklung befinden.

Die Spulenkerne, der aus dem Stand der Technik bekannten Magnetlagervorrichtungen sind meist geblecht ausgestaltet. Das bedeutet, dass mehrere Bleche in Form der Spulenkerne gegeneinander isoliert in Umfangsrichtung gestapelt sind. Die geblechte Ausgestaltung der Spulenkerne verhindert Wirbelströme für Magnetfelder, welche in Blechrichtung verlaufen also Felder, welche in axialer Richtung dem Längsschenkel folgen und in radialer Richtung dem Querschenkel folgen.

Für Magnetfelder, welche seitlich, also in Umfangsrichtung aus den Blechen des Längsschenkels und des Querschenkels austreten, ist die Isolation der Bleche unwirksam und es treten somit trotzdem Wirbelströme auf, da diese Magnetfelder orthogonal durch die Bleche hindurchgehen.

Gerade bei Magnetlagervorrichtungen, welche einen grossen magnetischen Spalt, wobei der magnetische Spalt als Abstand zwischen der Stirnfläche des Polstücks und des magnetisch wirksamen Kerns des Rotors in radialer Richtung definiert ist, aufweisen, werden orthogonale Feldanteile nicht vernachlässigbar und erzeugen merkliche Wirbelstromverluste.

Im Kontext dieser Anmeldung ist mit einem grossen magnetischen Spalt ein magnetischer Spalt gemeint, welcher grösser als 1% des Durchmessers des magnetisch wirksamen Kerns in radialer Richtung ist. In manchen Fällen kann der magnetische Spalt grösser gleich 5% des Durchmessers des magnetisch wirksamen Kerns in radialer Richtung sein.

Ausgehend von diesem Stand der Technik ist es daher eine Aufgabe der Erfindung, eine Magnetlagervorrichtung zur berührungslos magnetischen Lagerung eines Rotors mit einem scheibenförmigen oder ringförmigen magnetisch wirksamen Kern vorzuschlagen, welche geringere Wirbelstromverluste als der bisherige Stand der Technik aufweist.

Ferner ist es eine Aufgabe der Erfindung, einen elektromagnetischen Drehantrieb mit einer solchen Magnetlagervorrichtung vorzuschlagen.

Der diese Aufgabe lösende Gegenstand der Erfindung ist durch die Merkmale des unabhängigen Patentanspruchs gekennzeichnet.

Erfindungsgemäss wird also eine Magnetlagervorrichtung vorgeschlagen, zur berührungslosen magnetischen Lagerung eines Rotors, der einen scheibenförmigen oder ringförmigen magnetisch wirksamen Kern aufweist, wobei die Magnetlagervorrichtung einen Stator mit einer becherförmigen Ausnehmung aufweist, die an einem axialen Ende des Stators angeordnet ist, und in die der Rotor einsetzbar ist, wobei der Stator eine Mehrzahl von Spulenkernen aufweist, von denen jeder einen Längsschenkel und ein Polstück aufweist, wobei sich jeder Längsschenkel von einem ersten Ende in einer axialen Richtung bis zu einem zweiten Ende erstreckt, wobei am zweiten Ende eine Kontaktfläche angeordnet ist, wobei sich jedes Polstück von der Kontaktfläche zumindest teilweise in einer radialer Richtung bis zu einer Stirnfläche erstreckt, wobei die radiale Richtung senkrecht zur axialen Richtung ist, wobei die Stirnfläche um die becherförmige Ausnehmung herum angeordnet sind, und wobei an jedem Längsschenkel mindestens eine konzentrierte Wicklung angeordnet ist, welche den jeweiligen Längsschenkel umgibt, wobei die Längsschenkel aus einem ersten Material gefertigt sind und die Polstücke aus einem zweiten Material gefertigt sind und wobei das erste Material und das zweite Material unterschiedlich sind.

Das von der erfindungsgemässen Magnetlagervorrichtung umfasste Polstück entspricht im Wesentlichen dem Querschenkel einer aus dem Stand der Technik bekannten Magnetlagervorrichtung oder eines elektromagnetischen Drehantriebs in der Tempelanordnung (auch als Tempelmotor bekannt).

Durch die zweiteilige Ausgestaltung des Spulenkerns, wobei das Polstück und der Längsschenkel aus unterschiedlichen Materialien bestehen, können für beide das ideale Material ausgewählt werden, um die Wirbelstromverluste zu reduzieren. Durch die Wahl von unterschiedlichen Materialien ist es möglich die Wirbelstromverluste dort, wo sie besonders gross sind zu reduzieren. Meist ist dies an den Polstücken der Fall.

Das heisst die Wahl eines idealen Materials für das Polstück führt zu einer Reduzierung der Wirbelstromverluste für den gesamten Spulenkern. Die Ausgestaltung mit unterschiedlichen Materialien birgt somit eine sehr flexible Anpassung der Spulenkerne an die entsprechenden Ansprüche der Magnetlagervorrichtung.

Gemäss einer bevorzugten Ausgestaltung ist jeder Längsschenkel geblecht aus Elementen hergestellt, wobei die Elemente in Umfangsrichtung des Stators gestapelt sind. Die geblechte Ausgestaltung sorgt für eine Reduktion der Wirbelstromverluste im Längsschenkel. Der Stator gibt hierbei die Umfangsrichtung vor und entlang dieser Richtung sind ringförmig eine Mehrzahl von Spulenkernen angeordnet.

Gerade bei solch einer geblechten Ausgestaltung ist die Wahl von unterschiedlichen Materialien für den Längsschenkel und das Polstück vorteilhaft um die Wirbelstromverluste dort, wo sie besonders gross sind zu reduzieren. Die meisten Wirbelstromverluste treten an den Polstücken auf, da der Abstand benachbarter Polstücke, insbesondere an deren, der becherförmigen Ausnehmung zugewandten Enden besonders klein ist. Der Grund hierfür ist, dass die Wirbelstromverluste vor allem aus orthogonalen Feldern, also Felder, welche die Elemente orthogonal durchdringen, resultieren. Solche orthogonalen Felder haben eben gerade bei kleinen Abständen der Polstücken zueinander die Möglichkeit in Umfangsrichtung vom Polstück eines ersten Spulenkerns zum Polstück eines zweiten, benachbarten Spulenkerns zu fliessen.

Somit kann durch eine geeignete Wahl des zweiten Materials, welches nicht dem Material der geblechten Elemente des Längsschenkels entspricht, eine effektive Reduktion der Wirbelstromverluste dort erreicht werden, wo diese am stärksten auftreten.

Ebenso ist es möglich, dass der Längsschenkel aus einem Vollmaterial ausgestaltet ist.

Dabei ist es bevorzugt, dass das erste Material ein Elektroblech ist. Unter einem Elektroblech wird nach der allgemeinen Definition ein weichmagnetischer Werkstoff für Magnetkerne verstanden. Als weichmagnetische Werkstoffe bezeichnet man üblicherweise Werkstoffe, welche eine tiefe Koerzitivfeldstärke aufweisen. Die Koerzitivfeldstärke ist diejenige magnetische Feldstärke, die man benötigt, um einen Stoff zu entmagnetisieren. Im Rahmen dieser Anmeldung wird unter einem weichmagnetischen Werkstoff ein Material verstanden, welches eine Koerzitivfeldstärke, genauer gesagt eine Koerzitivfeldstärke der magnetischen Polarisation aufweist, die weniger als 2'000 A/m beträgt.

Ebenso besteht die Möglichkeit Mu-Metall für das erste Material zu verwenden. Wird der Längsschenkel aus einem Vollmaterial ausgestaltet, so werden Metalle oder Metallverbindungen, wie z.B. FeSi bevorzugt. Ebenso ist es möglich, die Polstücke geblecht auszugestalten.

Ferner ist es bevorzugt, dass das zweite Material ein Pulververbundwerkstoff ist, insbesondere ein weichmagnetischer Pulververbundwerkstoff. Dabei können diese, im Englischen "Soft Magnetic Composite (SMC)" genannten Werkstoffe hochreine Eisenpulver mit einer speziellen Oberflächenbeschichtung sein. Die spezielle Oberflächenbeschichtung ist in diesem Fall elektrisch isolierend. SMCs sind vor allem für ihre Anwendung bei der Führung von hochfrequenten magnetischen Feldern (Frequenz >> 1 kHz) bekannt. Eine Anwendung bei niedrigeren Frequenzen ist allerdings bisher nicht üblich. Mit einer niedrigeren Frequenz sind Frequenzen gemeint die grösser als 65 Hz sind. Erst ab einer Frequenz des Magnetfelds grösser als 65 Hz werden Wirbelstromverluste begünstigt.

Weitere Vorteile der weichmagnetischen Pulververbundwerkstoff sind, dass sie eine sehr gute Fähigkeit zur dreidimensionalen Flussführung aufweisen und einen hohen elektrischen Widerstand und hohe Permeabilität besitzen und somit praktisch keine Wirbelstromverluste aufweisen. Gerade die Fähigkeit zur dreidimensionalen Flussführung, ohne dass dabei hohe Wirbelstromverluste erzeugt werden, sorgt dafür, dass weichmagnetische Pulververbundwerkstoffe als das zweite Material verwendet werden.

Da weichmagnetische Pulververbundwerkstoffe vergleichsweise hohe Hystereseverluste aufweisen, wird in bevorzugten Ausführungsformen weichmagnetischer Pulververbundwerkstoff nur zur Herstellung der Polstücke verwendet. Dadurch findet sich ein ausgewogenes Mittel zwischen der Reduktion der Wirbelstromverluste an der Stelle an welchen sie besonders gross sind, zum unerwünschten Einfluss des Pulververbundwerkstoffs auf den Magnetkreis. Ein weiterer Grund für die Verwendung von Pulververbundwerkstoff nur für das Polstück ist, dass dieser an für sich ein sprödes, gesintertes Material mit unbekanntem Alterungsprozess ist, wodurch sich die Lebensdauer des Polstücks und somit der Magnetlagervorrichtung herabsetzen kann.

Es ist natürlich auch möglich, die Längsschenkel aus einem weichmagnetischen Pulververbundwerkstoff herzustellen, während die Polstücke aus einem anderen Material gefertigt werden.

Das Anbringen eines Polstücks an einen Längsschenkel kann durch mehrere mögliche Fügearten erfolgen. Diese umfassen unter anderem eine kraftschlüssige Fügeart, wie z.B. Klemmen oder Crimpen, eine formschlüssige Fügeart, wie z.B. Schrauben oder Stecken oder eine stoffschlüssige Verbindung, wie z.B. Kleben. Ebenso ist es möglich, die Verbindung zwischen Längsschenkel und Polstück über Nut-Feder-Verbindungen und/oder Spundungen, wie z.B. Zapfen, Zinken oder Schwalbenschwanzverbindungen herzustellen. In einer bevorzugten Ausführungsform wird die stoffschlüssige Fügeart durch Kleben umgesetzt. Ein Vorteil hierbei ist, dass der Klebstoff im gleichen Festigkeitsbereich liegt, wie Pulverkompositwerkstoff. Dies hat den Vorteil gegenüber Klemmen oder Schrauben, dass keine Spannungsüberhöhungen im Pulverkompositwerkstoff auftreten. Hingehen haben die kraftschlüssigen und formschlüssigen Fügearten den Vorteil, dass diese eine geringe Defektanfälligkeit, die durch z.B. Alterung oder Fehler beim Verkleben auftreten, aufweisen.

In einer bevorzugten Ausgestaltung ist die Kontaktfläche planar ausgestaltet und an einer Fläche des Längsschenkels angeordnet, die senkrecht auf der radialen Richtung steht.

Besonders bevorzugt ist hierbei, dass sich die Kontaktfläche in der Nähe des zweiten Endes des Längsschenkels befindet.

In einer anderen bevorzugten Ausgestaltung ist die Kontaktfläche an einer Fläche des Längsschenkel angeordnet, die senkrecht auf der axialen Richtung steht. Besonders bevorzugt ist hierbei die Kontaktfläche am zweiten Ende des Längsschenkels angeordnet.

Gemäss einer anderen bevorzugten Ausgestaltung ist die Kontaktfläche gewinkelt ausgestaltet. Gewinkelt kann in diesem Zusammenhang bedeuten, dass die Kontaktfläche in der Form eines "L" ausgestaltet ist und sich ein Bereich der Kontaktfläche in axialer Richtung und ein Bereich in radialer Richtung erstreckt.

Im Hinblick auf die stoffschlüssige Fügeart birgt diese Ausgestaltung einen weiteren Vorteil. Durch die gewinkelte Ausgestaltung der Kontaktfläche wird die verfügbare Klebefläche im Vergleich zu den nicht gewinkelten Ausgestaltungen der Kontaktfläche vergrössert.

Des Weiteren wird durch diese Ausgestaltung die Zugbelastung durch magnetische Kräfte an den Polstücken verringert.

Gemäss einer weiteren bevorzugten Ausführungsform weist die Kontaktfläche zwei Teilflächen auf, wobei die erste Teilfläche senkrecht auf der axialen Richtung steht und die zweite Teilfläche senkrecht auf der radialen Richtung steht.

In einer bevorzugten Ausgestaltung ist das Polstück gewinkelt ausgestaltet, wobei das Polstück zwei Teilstücke umfasst, von denen sich eines in radialer Richtung erstreckt und das andere in axialer Richtung.

In einer bevorzugten Ausgestaltung weist der Spulenkern an einem axial oberen Ende eine Abrundung auf, welche den Spulenkern aus der axialen Richtung in die radiale Richtung umlenkt. Als Beispiel, im Fall eines L-Förmigen Spulenkerns, wobei das lange Stück des "L" durch den Längsschenkel und das kurze Stück des "L" durch das Polstück gebildet wird, ist die von der becherförmigen Ausnehmung betrachtet radial aussen liegende Kante des Polstücks, die sich in der radialen Ebene in Umfangsrichtung erstreckt, abgerundet ausgestaltet. Diese Ausgestaltung hat den Vorteil, dass sie geringere Wirbelstromverluste aufweist und auch im Hinblick auf die Konstruktion einfacher umzusetzen ist. Diese Ausgestaltung kann mit allen Ausgestaltungen der Kontaktfläche, der Stirnfläche und der Polstücke bzw. Längsschenkel umgesetzt werden.

In einer bevorzugten Ausgestaltung ist die Stirnfläche des Polstücks als gekrümmte Fläche ausgestaltet. Besonders bevorzugt ist dabei, dass die Rundung der Stirnfläche koaxial zur becherförmigen Ausnehmung ausgestaltet ist. In anderen Worten, die Stirnfläche des Polstücks ist ein Segment einer Zylinderoberfläche, wobei die Mittelachse dieses Zylinders mit der Mittelachse der becherförmigen Ausnehmung zusammenfällt und dessen Radius grösser ist als der der becherförmigen Ausnehmung, sodass die Stirnfläche nicht in die becherförmige Ausnehmung hineinragt.

In einer bevorzugten Ausgestaltung ist die Stirnfläche bezüglich der Umfangrichtung breiter ausgestaltet als die maximale Erstreckung der Kontaktfläche in Umfangsrichtung. Das bedeutet, dass eine der beiden Kanten der Stirnfläche, welche sich in Umfangsrichtung erstrecken, länger ist als eine der Kanten der Kontaktfläche, welche sich in Umfangsrichtung erstrecken. Ist die Stirnfläche als gekrümmte Fläche ausgestaltet, so ist die Länge einer der Kreisbögen des Segments der Zylinderoberfläche in der radialen Ebene grösser als die Länge einer der Kanten der Kontaktfläche, welche sich in Umfangsrichtung erstrecken.

Diese Verbreiterung der Stirnfläche in Umfangrichtung hat den Vorteil, da dadurch die magnetische Funktionalität begünstigt wird. Zum Beispiel können die passive Steifigkeit und die aktiven Lagerkräfte verbessert werden.

Gemäss einer besonders bevorzugten Ausführungsform ist der Stator der Magnetlagervorrichtung zur Erzeugung eines Drehmoments ausgestaltet, mit welchem der Rotor berührungslos magnetisch zur Rotation um die axiale Richtung antreibbar ist.

Hierbei ist Stator als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation um eine Solldrehachse bewirkt, und welches zum anderen eine beliebig einstellbare Querkraft auf den Rotor ausübt, sodass dessen radiale Position aktiv steuerbar bzw. regelbar ist.

Gerade im Hinblick auf die Ausführungsform, bei welcher die Magnetlagervorrichtung zur Erzeugung eines Drehmoments ausgestaltet ist, ist die Ausgestaltung mit der breiteren Stirnfläche von Vorteil, da dadurch die magnetische Funktionalität begünstigt wird. Zum Beispiel kann ein erhöhtes Drehmoment erzeugt werden oder aber die passive Steifigkeit oder die aktiven Lagerkräfte verbessert werden.

Durch die Erfindung wird ferner ein elektromagnetischer Drehantrieb, der als Tempelmotor ausgestaltet ist vorgeschlagen, wobei der elektromagnetische Drehantrieb eine erfindungsgemässe Magnetlagervorrichtung umfasst, sowie einen Rotor mit einem scheibenförmigen oder ringförmigen magnetisch wirksamen Kern, wobei der Rotor in die becherförmige Ausnehmung einsetzbar ist, und wobei der Rotor als Rotor des elektromagnetischen Drehantriebs ausgestaltet ist.

Solche elektromagnetischen Drehantriebe sind auch unter dem Begriff des lagerlosen Motors bekannt. Mit dem Begriff lagerloser Motor ist dabei ein elektromagnetischer Drehantrieb gemeint, bei welchem der Rotor vollkommen magnetisch bezüglich des Stators gelagert ist, wobei keine separaten magnetischen Lager vorgesehen sind.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der Zeichnung zeigen:
- Fig. 1:: eine perspektivische Darstellung eines Ausführungsbeispiels einer erfindungsgemässen Magnetlagervorrichtung,
- Fig. 2:: eine perspektivische Darstellung eines einzelnen Spulenkerns der Magnetlagerlagervorrichtung aus Fig. 1, sowie die Draufsicht auf diesen,
- Fig. 3: schematische Schnittdarstellungen mehrerer Ausführungsbeispiele eines Spulenkerns
- Fig. 4:: eine perspektivische Darstellung einer ersten Variante des Ausführungsbeispiels eines Spulenkerns aus Fig. 3 b), sowie die Draufsicht auf diesen,
- Fig. 5:: eine perspektivische Darstellung einer zweiten Variante des Ausführungsbeispiels eines Spulenkerns aus Fig. 3 b), sowie die Draufsicht auf diesen,
- Fig. 6:: eine schematische Schnittdarstellung eines weiteren Ausführungsbeispiels eines Spulenkerns,
- Fig. 7:: eine perspektivische Darstellung einer Variante des Ausführungsbeispielseines Spulenkerns aus Fig. 6, sowie die Draufsicht auf diesen,
- Fig. 8:: eine schematische Schnittdarstellung eines Ausschnitts eines Ausführungsbeispiels eines Spulenkerns zur Verdeutlichung der wirkenden Zugkräfte,
- Fig. 9:: eine perspektivische Darstellung eines weiteren Ausführungsbeispiels eines Spulenkerns,
- Fig. 10:: eine perspektivische Darstellung eines weiteren Ausführungsbeispiels einer erfindungsgemässen Magnetlagervorrichtung,
- Fig. 11:: eine schematische Schnittdarstellung für eine Ausgestaltung eines Statorgehäuses,
- Fig. 12:: eine schematische Schnittdarstellung eines aus dem Stand der Technik bekannten Spulenkerns, sowie die Draufsicht auf diesen.

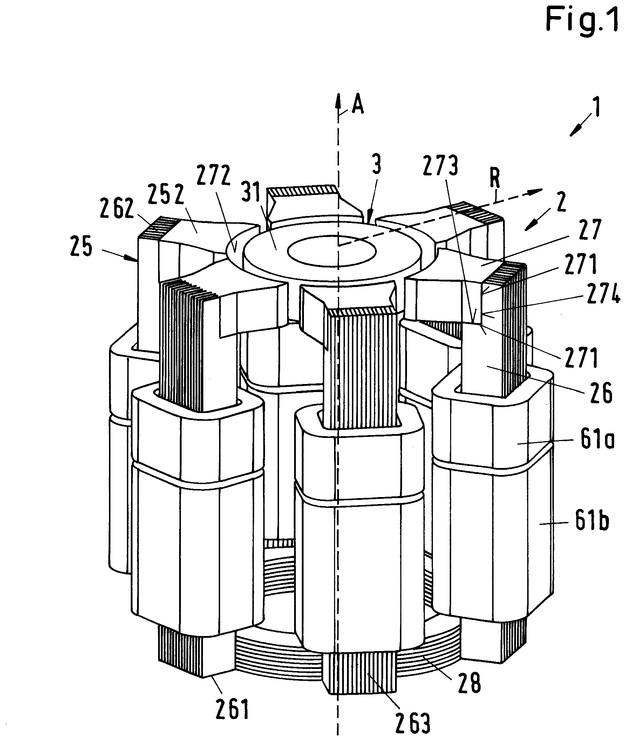

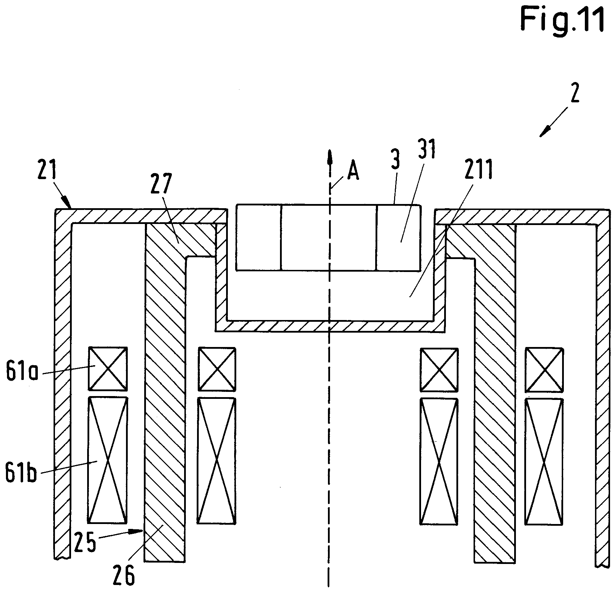

Fig. 1 zeigt eine perspektivische Darstellung eines Ausführungsbeispiels einer erfindungsgemässen Magnetlagervorrichtung, die gesamthaft mit dem Bezugszeichen 1 bezeichnet ist. Die Magnetlagervorrichtung 1 ist für die berührungslos magnetische Lagerung eines Rotors 3 ausgestaltet, der einen scheibenförmigen oder ringförmigen magnetisch wirksamen Kern 31 umfasst. Die Magnetlagervorrichtung 1 ist gemäss der Tempelbauweise ausgestaltet und umfasst einen Stator 2. Üblicherweise umfasst der Stator 2 ein Statorgehäuse 21 (Fig. 11), welches in Fig. 1 aus Gründen der besseren Übersicht allerdings nicht dargestellt ist. An einem axialen Ende des Statorgehäuses 21 (Fig. 11) ist eine becherförmige Ausnehmung 211 (Fig. 11) vorgesehen in die der Rotor 3 einsetzbar ist. Der Rotor 3 ist zur Rotation um eine Solldrehachse ausgestaltet. Diese Solldrehachse definiert eine axiale Richtung A. Üblicherweise stimmt die Mittelachse des Stators 2, welche sich in der axialen Richtung A erstreckt, mit der Solldrehachse überein. Die Solldrehachse bezeichnet dabei diejenige Achse, um welche sich der Rotor 3 im Betriebszustand dreht, wenn sich der Rotor 3 bezüglich des Stators 2 in einer zentrierten und unverkippten Lage befindet, so wie dies in Fig. 1 dargestellt ist.

Der Stator 2 weist eine Mehrzahl von Spulenkernen 25 - hier sechs Spulenkerne 25 - auf, von denen jeder einen Längsschenkel 26 und ein Polstück 27 aufweist. Jeder Längsschenkel 26 erstreckt sich von einem ersten Ende 261 in axialer Richtung A bis zu einem zweiten Ende 262, wobei am zweiten Ende 262 eine Kontaktfläche 271 angeordnet ist, von welcher sich jedes Polstück 27 zumindest teilweise in einer radialer Richtung R bis zu einer Stirnfläche 272 erstreckt. Dabei sind die Stirnflächen 272 dem Rotor 3 zugewandt und um diesen herum angeordnet. In anderen Worten, die Polstücke 27 der Spulenkerne 25 sind derart angeordnet, dass die Stirnflächen 272 der Polstücke 27 um die becherförmige Ausnehmung 211 (Fig. 11) herum angeordnet sind. Die Spulenkerne 25 des Stators 2 sind äquidistant auf einer Kreislinie angeordnet, sodass die Stirnflächen 272 den magnetisch wirksamen Kern 31 des Rotors 3 umgeben, wenn der Rotor 3 in die becherförmige Ausnehmung 211 (Fig. 11) eingesetzt ist.

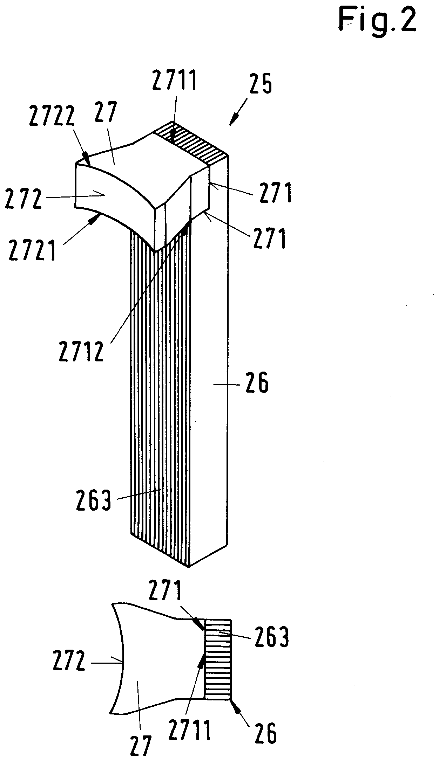

Zum besseren Verständnis ist in Fig. 2 eine perspektivische Darstellung eines einzelnen Spulenkerns 25 der Magnetlagerlagervorrichtung 1 aus Fig. 1, sowie die Draufsicht auf diesen, dargestellt.

An jedem Längsschenkel 26 ist mindestens eine konzentrierte Wicklung 61 angeordnet, welche den jeweiligen Längsschenkel 26 umgibt. In anderen Ausführungsformen können an den Längsschenkeln 26 auch mehr als eine konzentrierte Wicklung angeordnet sein. Beispielsweise gibt es Ausführungsbeispiele, bei denen an jedem der Längsschenkel 26 jeweils genau zwei konzentrierte Wicklungen vorgesehen sind, von denen jede den jeweiligen Längsschenkel 26 umgibt, wobei die beiden auf dem gleichen Längsschenkel 26 angeordneten Wicklungen bezüglich der axialen Richtung A benachbart zueinander angeordnet sind.

Die konzentrierten Wicklungen 61 dienen dazu, elektromagnetische Felder zu erzeugen, mit welchen der Rotor 3 berührungslos magnetisch in der becherförmigen Ausnehmung 211 (Fig. 11) lagerbar ist.

In diesem Ausführungsbeispiel sind die Längsschenkel 26 geblecht aus Elementen 263 hergestellt, wobei die Elemente 263 in Umfangsrichtung des Stators 2 gestapelt sind. Mit Umfangsrichtung ist die Richtung gemeint, welche senkrecht auf der radialen Richtung R und senkrecht auf der axialen Richtung A steht. Dabei sind die Längsschenkel 26 aus einem ersten Material gefertigt, hier aus einem Elektroblech und die Polstücke 27 aus einem zweiten Material gefertigt, wobei dieses hier ein Pulververbundwerkstoff ist, bevorzugt ein weichmagnetischer Pulververbundwerkstoff. Somit sind das erste Material und das zweite Material unterschiedlich.

Die Anzahl der Elemente 263 in allen Ausführungsbeispielen und Figuren ist rein exemplarisch zu verstehen. Die Anzahl kann grösser oder aber auch kleiner sein als dargestellt.

In diesem Ausführungsbeispiel ist die Kontaktfläche 271 gewinkelt ausgestaltet. Dabei weist die Kontaktfläche 271 zwei Teilflächen auf, wobei die erste Teilfläche 273 senkrecht auf der axialen Richtung steht und die zweite Teilfläche 274 senkrecht auf der radialen Richtung steht.

In diesem Ausführungsbeispiel ist die Stirnfläche 272 des Polstücks 27 als gekrümmte Fläche ausgestaltet. Die Rundung der Stirnfläche 272 ist koaxial zur becherförmigen Ausnehmung 211 (Fig. 11) ausgestaltet. Hierbei ist die Stirnfläche 272 bezüglich der Umfangrichtung breiter ausgestaltet als die maximale Erstreckung der Kontaktfläche 271 in Umfangsrichtung.

In anderen Worten, die Stirnfläche 272 des Polstücks 27 ist ein Segment einer Zylinderoberfläche, wobei die Mittelachse dieses Zylinders mit der Mittelachse der becherförmigen Ausnehmung 211 (Fig. 11), in diesem Ausführungsbeispiel die Achse der axialen Richtung A, zusammenfällt und dessen Radius grösser ist als der der becherförmigen Ausnehmung 211 (Fig. 11), sodass die Stirnfläche 272 nicht in die becherförmige Ausnehmung 211 (Fig. 11) hineinragt.

In einer bevorzugten Ausgestaltung ist die Stirnfläche 272 bezüglich der Umfangrichtung breiter ausgestaltet als die maximale Erstreckung der Kontaktfläche 271 in Umfangsrichtung. Das bedeutet, dass eine der beiden Kanten 2721 oder 2722 der Stirnfläche 272, welche sich in Umfangsrichtung erstrecken, länger ist als eine der Kanten 2711 oder 2712 der Kontaktfläche 271, welche sich in Umfangsrichtung erstrecken. Ist die Stirnfläche 272 als gekrümmte Fläche ausgestaltet, so ist die Länge einer der Kreisbögen 2721 oder 2722 des Segments der Zylinderoberfläche in einer radialen Ebene E grösser als die Länge einer der Kanten 2711 oder 2712 der Kontaktfläche 271, welche sich in Umfangsrichtung erstrecken. Die radiale Ebene ist in Fig. 1 durch die Linie einer radialen Richtung R angedeutet, die senkrecht auf der axialen Richtung A steht. Die radiale Ebene E ist diejenige Ebene, welche senkrecht auf der axialen Richtung A steht und eine radiale Richtung R enthält. Die radiale Ebene E ist diejenige Ebene, in welcher der magnetisch wirksame Kern 31 des Rotors 3 im Betriebszustand zwischen den Stirnflächen 272 im Stator 2 aktiv magnetisch gelagert ist. Wenn der Rotor 3 nicht verkippt und in axialer Richtung A nicht ausgelenkt ist, liegt die magnetische Mittelebene in der radialen Ebene E. Die radiale Ebene E definiert die x-y-Ebene eines kartesischen Koordinatensystems, dessen z-Achse in axialer Richtung A verläuft.

Mit der radialen Position des magnetisch wirksamen Kerns 31 bzw. des Rotors 3 wird die Lage des Rotors 3 in der radialen Ebene E bezeichnet.

Gemäss einer speziell bevorzugten Ausgestaltung ist der Stator 2 so ausgestaltet, dass er zusätzlich zur berührungslos magnetischen Lagerung des Rotors 3 auch ein Drehmoment auf den Rotor 3 bzw. den magnetisch wirksamen Kern 31 des Rotors 3 ausüben kann, welches den Rotor 3 zu einer Rotation um die Solldrehachse antreibt. Das heisst, bei dieser bevorzugten Ausgestaltung ist der Rotor 3 zur Rotation um die axiale Richtung A antreibbar.

Die bereits erwähnte Verbreiterung der Stirnfläche 272 in Umfangrichtung hat den Vorteil, dass dadurch die magnetische Funktionalität begünstigt wird. Zum Beispiel können die passive Steifigkeit und die aktiven Lagerkräfte verbessert werden. Im Hinblick auf die Ausführungsform, bei welcher die Magnetlagervorrichtung 1 zur Erzeugung eines Drehmoments ausgestaltet ist, ergibt sich zudem der Vorteil, dass ein erhöhtes Drehmoment erzeugt werden kann.

Bei dieser Ausgestaltung erzeugen die konzentrierten Wicklungen 61 also elektromagnetische Drehfelder, mit denen der Rotor 3 sowohl berührungslos magnetisch bezüglich des Stators 2 lagerbar ist als auch berührungslos zur Rotation um die axiale Richtung A antreibbar ist.

Es versteht sich, dass die Anzahl von sechs Spulenkernen 25 zwar bevorzugt, aber nur beispielhaft zu verstehen ist. Es sind natürlich auch solche Ausgestaltungen möglich, bei denen der Stator 2 weniger als sechs, z. B. fünf oder vier oder drei Spulenkerne 25 aufweist, oder solche Ausgestaltungen, bei denen der Stator 2 mehr als sechs, z. B. sieben oder acht oder neun Spulenkerne 25 aufweist oder eine beliebige grössere Anzahl von Spulenkernen 25.

Der Rotor 3 umfasst den magnetisch wirksamen Kern 31, welcher ring- oder scheibenförmig ausgestaltet ist. Der magnetisch wirksame Kern 31 ist gemäss der Darstellung in Fig. 1 als Ring ausgestaltet und definiert eine magnetische Mittelebene. Alternativ kann der magnetisch wirksame Kern 31 auch als Scheibe ausgestaltet sein. In der Regel ist bei einem scheibenförmigen oder ringförmigen magnetisch wirksamen Kern 31 die magnetische Mittelebene die geometrische Mittelebene des magnetisch wirksamen Kerns 31 des Rotors 3, die senkrecht zur axialen Richtung A liegt. Im Betriebszustand ist der magnetisch wirksame Kern 31 in der radialen Ebene E gelagert, welche senkrecht auf der axialen Richtung A steht.

Da es für das Verständnis der Erfindung ausreichend ist, ist in der Zeichnung in der Fig. 1 von dem Rotor 3 nur der magnetisch wirksame Kern 31 dargestellt. Es versteht sich, dass der Rotor 3 natürlich auch noch weitere Komponenten umfassen kann wie beispielsweise Ummantelungen oder Kapselungen, die vorzugsweise aus einem Kunststoff hergestellt sind, oder aus einem Metall oder aus einer Metalllegierung oder aus einer Keramik bzw. einem keramischen Werkstoff. Ferner kann der Rotor 3 auch Flügel zum Mischen, Rühren oder Pumpen von Fluiden oder sonstige Komponenten umfassen.

Wenn der Rotor 3 in die becherförmige Ausnehmung 211 (Fig. 11) eingesetzt ist, wird der Rotor 3 und insbesondere der magnetisch wirksame Kern 31 des Rotors 3 von den radial aussenliegend angeordneten Stirnflächen 272 der Polstücke 27 der Spulenkerne 25 des Stators 2 umgeben. Die Polstücke 27 bilden somit eine Mehrzahl von ausgeprägten Statorpolen- hier sechs Statorpole.

Wenn sich der magnetisch wirksame Kern 31 des Rotors 3 während des Betriebs in seiner Soll-Position befindet, so ist der magnetisch wirksame Kern 31 zwischen den Stirnflächen 272 der Polstücke 27 zentriert. Die konzentrierten Wicklungen 61 sind darstellungsgemäss unterhalb der radialen Ebene E angeordnet und so ausgerichtet, dass ihre Spulenachsen in axialer Richtung A verlaufen.

Alle ersten Enden 261 der Längsschenkel 26 - also die darstellungsgemäss (Fig. 1) unteren Enden 261 - sind durch einen Rückschluss 28 miteinander verbunden. Der Rückschluss 28 ist vorzugsweise ringförmig ausgestaltet. Dabei sind solche Ausgestaltungen möglich (siehe z. B. Fig. 1), bei welchen sich der Rückschluss 28 radial innenliegend entlang aller ersten Enden 261 der Längsschenkel 26 erstreckt.

Um die für die magnetische Lagerung des Rotors 3 und optional zur Erzeugung eines Drehmoments auf den Rotor 3 notwendigen elektromagnetischen Felder zu erzeugen, tragen die Längsschenkel 26 der Spulenkerne 25 die als konzentrierte Wicklungen 61 ausgestalteten Wicklungen.

Mit diesen konzentrierten Wicklungen 61 werden im Betriebszustand diejenigen elektromagnetischen Drehfelder erzeugt, mit welchen eine beliebig einstellbare Querkraft in radialer Richtung auf den Rotor 3 ausübbar ist, sodass die radiale Position des Rotors 3, also seine Position in der zur axialen Richtung A senkrechten radialen Ebene E, aktiv steuerbar bzw. regelbar ist. Optional wird mit diesen elektromagnetischen Drehfeldern zusätzlich ein Drehmoment auf den Rotor 3 bewirkt.

Mit dem "magnetisch wirksamen Kern 31" des Rotors 3 ist derjenige Bereich des Rotors 3 gemeint, welcher für die Erzeugung der magnetischen Lagerkräfte und optional für die Drehmomentbildung magnetisch mit dem Stator 2 zusammenwirkt.

Wie bereits erwähnt, ist der magnetisch wirksame Kern 31 bei diesem Ausführungsbeispiel ringförmig ausgestaltet. Ferner ist der magnetisch wirksame Kern 31 permanentmagnetisch ausgestaltet. Dazu kann der magnetisch wirksame Kern 31 mindestens einen Permanentmagneten, aber auch mehrere Permanentmagnete umfassen oder - wie im hier beschriebenen Ausführungsbeispiel - vollständig aus einem permanentmagnetischen Material bestehen, sodass der magnetisch wirksame Kern 31 der Permanentmagnet ist. Der magnetisch wirksame Kern 31 ist beispielsweise in radialer Richtung magnetisiert.

Als Permanentmagnete bezeichnet man üblicherweise solche ferromagnetischen oder ferrimagnetischen Werkstoffe, die hartmagnetisch sind, also eine hohe Koerzitivfeldstärke aufweisen. Die Koerzitivfeldstärke ist diejenige magnetische Feldstärke, die man benötigt, um einen Stoff zu entmagnetisieren. Im Rahmen dieser Anmeldung wird unter einem Permanentmagneten ein Werkstoff bzw. ein Material verstanden, der eine Koerzitivfeldstärke, genauer gesagt eine Koerzitivfeldstärke der magnetischen Polarisation aufweist, die mehr als 10'000 A/m beträgt.

Es sind auch solche Ausgestaltungen möglich, bei denen der magnetisch wirksame Kern 31 permanentmagnetfrei, also ohne Permanentmagnete ausgestaltet ist. Der Rotor 3 ist dann z.B. als Reluktanzläufer ausgestaltet. Der magnetisch wirksame Kern 31 des Rotors 3 besteht dann beispielsweise aus einem weichmagnetischen Material. Geeignete weichmagnetische Materialien für den magnetisch wirksamen Kern 31 sind beispielsweise ferromagnetische oder ferrimagnetische Materialien, also insbesondere Eisen, Nickel-Eisen, Kobalt-Eisen, Silizium-Eisen, Mu-Metall.

Ferner sind Ausgestaltungen möglich, bei denen der magnetisch wirksame Kern 31 des Rotors 3 sowohl ferromagnetische Materialien als auch permanentmagnetische Materialien umfasst. Beispielsweise können Permanentmagnete in einen ferromagnetischen Grundkörper eingelegt bzw. eingesetzt werden. Solche Ausgestaltungen sind z.B. vorteilhaft, wenn man bei grossen Rotoren die Kosten durch Einsparen von permanentmagnetischem Material reduzieren will.

Auch sind Ausgestaltungen möglich, bei denen der Rotor nach dem Prinzip eines Käfigläufers ausgestaltet ist.

Der Stator 2 ist frei von Permanentmagneten. Mit dieser Bezeichnung, dass der Stator 2 "frei von Permanentmagneten" ausgestaltet ist, soll im Rahmen dieser Anmeldung verstanden werden, dass der Stator 2 keine Permanentmagnete umfasst, die einen wesentlichen Beitrag zum Antriebsfeld zum Antreiben der Rotation des Rotors 3 oder zur Generierung der magnetischen Lagerkräfte für den Rotor 3 leisten. Der generierte magnetische Fluss durch den Stator 2 für den Antrieb und die Lagerung des Rotors 3 umfasst also keinen permanentmagnetisch erregten Fluss.

Es ist natürlich möglich, dass der Rotor 3 und/oder der Stator 2 andere Magnete bzw. Permanentmagnete umfassen, beispielsweise in Sensoren, welche z. B. der Erfassung der Winkelstellung des Rotors dienen, oder die sonst einen Zweck erfüllen, der nichts mit dem Generieren des magnetischen Flusses für den Antrieb und die Lagerung des Rotors 3 zu tun hat.

Die Bezeichnung "frei von Permanentmagneten" bezieht sich also nur auf die Generierung des magnetischen Flusses für den Antrieb und die Lagerung des Rotors 3 durch den Stator 2. In anderen Worten, der Stator 2 weist keine Permanentmagnete auf, welche einen Beitrag zum magnetischen Fluss leisten, mittels welchem der Rotor 3 angetrieben und magnetisch gelagert wird.

Es ist aber trotzdem möglich, dass der magnetische Fluss für den Antrieb und die Lagerung des Rotors 3 einen permanentmagnetischen Fluss umfasst, aber dieser wird dann nur vom Rotor 3 selbst generiert. Dies wäre der Fall, wenn der Rotor 3 selbst einen Permanentmagneten umfassen sollte.

Der ringförmige Rückschluss 28 kann aus einem weichmagnetischen Material gefertigt sein, weil es als Flussleitelemente zur Führung des magnetischen Flusses dient.

Geeignete weichmagnetische Materialien den Rückschluss 28 sind beispielsweise ferromagnetische oder ferrimagnetische Materialien, also insbesondere Eisen, Nickel-Eisen, Kobalt-Eisen Silizium-Eisen oder Mu-Metall. Hierbei ist für den Stator 2 eine Ausgestaltung als Statorblechpaket bevorzugt, bei welcher der Rückschluss 28, geblecht ausgestaltet ist, das heisst er besteht aus mehreren dünnen Blechelementen, die parallel zueinander in axialer Richtung A aufeinandergestapelt sind. Alle Rückschlusselemente sind identisch ausgestaltet, hier also jeweils im Wesentlichen ringförmig und auch mit der gleichen Dicke. Somit ist der Rückschluss 28 selbst im Wesentlichen ringförmig ausgestaltet und erstreckt sich im zusammengesetzten Zustand radial innenliegend entlang der ersten Enden 261 der Längsschenkel 26.

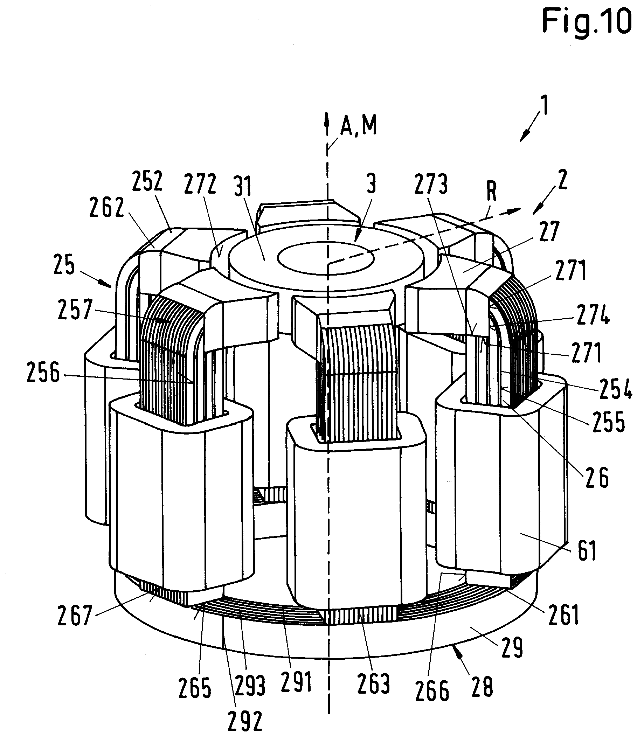

Ebenso sind Ausgestaltungen denkbar bei welchen als Rückschluss 28 ein sog. Ringbandkern verwendet wird. Solch ein Rückschluss 28 ist in dem Ausführungsbeispiel in Fig. 10 dargestellt. Dabei handelt es sich um ein aufgewickeltes Band aus Elektroblech. Bevorzugt wird hierbei kornorientiertes Elektroblech benutzt. Ringbandkerne sind im Stand der Technik vor allem bei der Verwendung für Transformatoren, Übertrager, Induktivitäten bekannt aber nicht für Lagervorrichtungen und vor allem nicht für elektromagnetische Drehantriebe.

Ferner ist es möglich, dass der Rückschluss 28 aus gepressten und anschliessend gesinterten Körnern der genannten Materialien bestehen. Die metallischen Körner sind dabei vorzugsweise in eine Kunststoffmatrix eingebettet, sodass sie zumindest teilweise gegeneinander isoliert sind, wodurch sich Wirbelstromverluste minimieren lassen. Es sind also für den Stator auch weichmagnetische Verbundwerkstoffe geeignet, welche aus elektrisch isolierten und zusammengepressten Metallpartikeln bestehen. Insbesondere können diese weichmagnetischen Verbundwerkstoffe, die auch als SMC (Soft Magnetic Composites) bezeichnet werden, aus Eisenpulverpartikeln bestehen, die mit einer elektrisch isolierenden Schicht beschichtet sind. Diese SMC werden dann mittels pulvermetallurgischer Verfahren zu der gewünschten Ausgestaltung geformt.

Während des Betriebs der Magnetlagervorrichtung 1 wirkt, der magnetisch wirksame Kern 31 des Rotors 3 mit dem Stator 2 zusammen, derart, dass der Rotor 3 berührungslos magnetisch bezüglich des Stators 2 lagerbar ist, und vorzugsweise zusätzlich berührungslos magnetisch in Rotation um die axiale Richtung A versetzt werden kann. Dabei ist es besonders vorteilhaft, dass dieselben Wicklungen 61 mit denen die magnetische Lagerung des Rotors 3 bewirkt wird, auch zur Erzeugung eines Drehmoments auf den Rotor 3 dienen. Vorzugsweise sind dann drei Freiheitsgrade des Rotors 3, nämlich seine Position in der radialen Ebene E und seine Rotation, aktiv regelbar. Bezüglich seiner axialen Auslenkung aus der radialen Ebene E in axialer Richtung A ist der magnetisch wirksame Kern 31 des Rotors 3 passiv magnetisch, das heisst nicht ansteuerbar, durch Reluktanzkräfte stabilisiert. Auch bezüglich der verbleibenden zwei Freiheitsgrade, nämlich Verkippungen bezüglich der zur Solldrehachse senkrechten radialen Ebene E ist der magnetisch wirksame Kern 31 des Rotors 3 ebenfalls passiv magnetisch stabilisiert. Der Rotor 3 ist also durch das Zusammenwirken des magnetisch wirksamen Kerns 31 mit den Spulenkernen 25 in axialer Richtung A sowie gegen Verkippungen (insgesamt drei Freiheitsgrade) passiv magnetisch gelagert oder passiv magnetisch stabilisiert und in der radialen Ebene (zwei Freiheitsgrade) aktiv magnetisch gelagert.

Wie dies allgemein üblich ist, bezeichnet auch im Rahmen dieser Anmeldung eine aktive magnetische Lagerung eine solche, die aktiv steuer- bzw. regelbar ist, beispielsweise über die mit den konzentrierten Wicklungen 61 generierten elektromagnetischen Felder. Eine passive magnetische Lagerung oder eine passive magnetische Stabilisierung bezeichnet eine solche, die nicht ansteuerbar bzw. regelbar ist. Die passive magnetische Lagerung oder Stabilisierung basiert beispielsweise auf Reluktanzkräften, welche den Rotor 3 bei einer Auslenkung aus seiner Soll-Position, also z. B. bei einer Verschiebung oder Auslenkung in axialer Richtung A oder bei einer Verkippung, wieder in seine Soll-Position bringen.

Bei der Magnetlagervorrichtung 1 wird im Unterschied zu klassischen Magnetlagern die magnetische Lagerung - und optional die Generierung eines auf den Rotor wirkenden Drehmoments - über elektromagnetische Drehfelder realisiert. Zur kombinierten Erzeugung der magnetischen Lagerkräfte und eines Drehmoments zum Rotieren des Rotors 3 um die axiale Richtung A ist es einerseits möglich - wie in Fig 1 gezeigt-, an jedem Längsschenkel 26 genau eine konzentrierte Wicklung 61 anzuordnen.

Andererseits sind auch Ausgestaltungen möglich, bei denen zur kombinierten Erzeugung der magnetischen Lagerkräfte und eines Drehmoments zum Rotieren des Rotors 3 zwei unterschiedliche Wicklungssysteme vorgesehen sind. Dazu sind dann beispielsweise an jedem Längsschenkel 26 jeweils genau zwei konzentrierte Wicklungen angeordnet, die bezüglich der axialen Richtung A benachbart zueinander angeordnet sind. Eine dieser beiden Wicklungen gehört zu dem ersten der beiden Wicklungssystem und die andere zu dem zweiten der beiden Wicklungssysteme.

Bei der in Fig. 1 dargestellten Ausführungsform mit genau einer konzentrierten Wicklung 61 an jedem Spulenkern 25 werden beispielsweise die in einer Kontrolleinheit jeweils ermittelten Werte für den für die Lagerung benötigten Strom und den für die Erzeugung des Drehmoments benötigten Strom rechnerisch -also z. B. mit Hilfe von Software - addiert bzw. überlagert. Der sich daraus ergebende Gesamtstrom wird dann in die jeweilige konzentrierte Wicklung 61 eingeprägt.

Ist der Stator 2 der erfindungsgemässen Magnetlagervorrichtung 1 zur Erzeugung eines Drehmoments ausgestaltet, so ist die Magnetlagervorrichtung 1 für einen elektromagnetischen Drehantrieb, der als Tempelmotor ausgestaltet ist, geeignet. Ebenso ist es möglich, dass die erfindungsgemässe Magnetlagervorrichtung 1 auch für andere Vorrichtungen wie z.B. Zentrifugalpumpen, Mischvorrichtungen zum Mischen fliessfähiger Substanzen, Rührvorrichtungen, beispielsweise zum Durchmischen eines Fluids in einem Tank, Lüfter oder auch Vorrichtungen zum Tragen und Rotieren von Wafern, beispielsweise in der Halbleiterfertigung, geeignet ist.

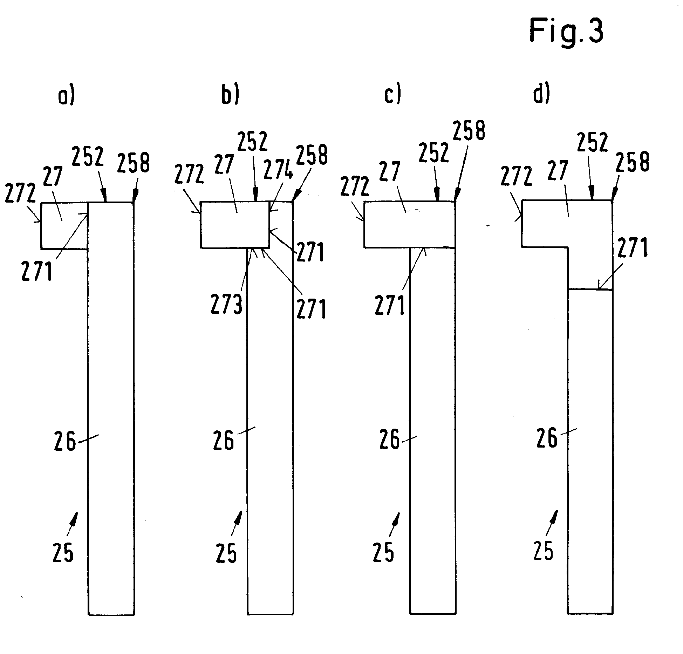

Fig. 3 zeigt schematische Schnittdarstellungen mehrerer Ausführungsbeispiele eines Spulenkerns 25.

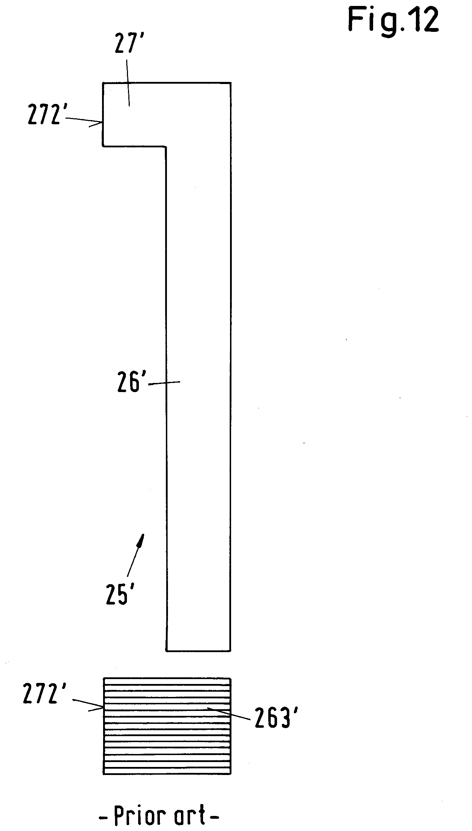

Fig. 12 zeigt zum Vergleich eine schematische Schnittdarstellung eines aus dem Stand der Technik bekannten Spulenkerns 25', sowie die Draufsicht auf diesen. Dabei ist der Spulenkern 25' einteilig ausgestaltet und der Längsschenkel 26' und das Polstück 27' bilden eine Einheit. Hierbei ist der komplette Spulenkern 25' geblecht aus Elementen 263' aufgebaut.

Für alle in Fig. 3 abgebildeten Ausführungsbeispiele eines Spulenkerns 25 soll vorzugsweise der Längsschenkel 26 geblecht aus Elementen 263 hergestellt sein, wobei die Elemente 263 in Umfangsrichtung des Stators 2 gestapelt sind, und wobei die Längsschenkel 26 aus Elektroblech gefertigt sind und die Polstücke 27 vorzugsweise aus einem Pulververbundwerkstoff, besonders bevorzugt einem weichmagnetischen Pulververbundwerkstoff gefertigt sind.

Fig 3 a) zeigt ein weiteres Ausführungsbeispiel eines Spulenkerns 25, bei welcher die Kontaktfläche 271 planar ausgestaltet ist und an einer Fläche des Längsschenkels 26 angeordnet ist, die senkrecht auf der radialen Richtung steht. Somit ist das Polstück 27 an einer Seitenfläche des Längsschenkel 26 angeordnet. In dieser Variante befindet sich das Polstück 27 am oberen zweiten Ende 262 des Längsschenkels 26.

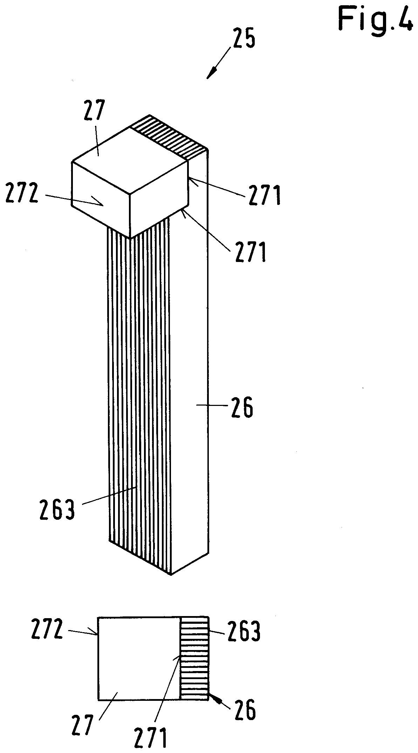

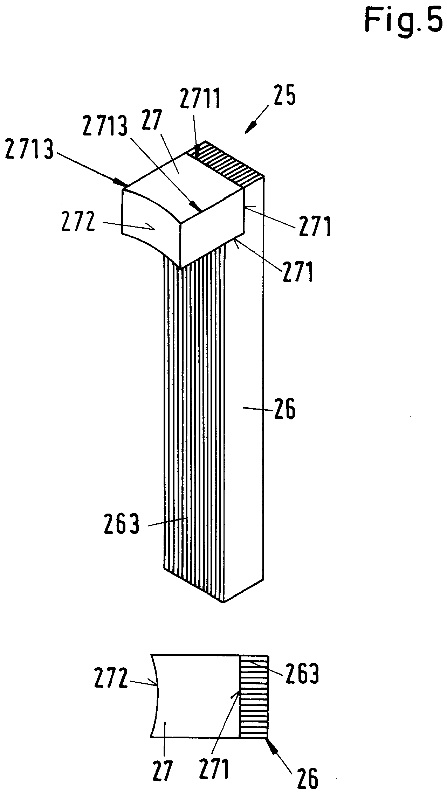

Fig. 3 b) zeigt ein weiteres Ausführungsbeispiel eines Spulenkerns 25, bei welcher die Kontaktfläche 271 gewinkelt ausgestaltet ist. Die Kontaktfläche 271 lässt sich somit in zwei Teilflächen unterteilen, wobei die erste Teilfläche 273 senkrecht auf der axialen Richtung steht und die zweite Teilfläche 274 senkrecht auf der radialen Richtung steht. Die Spulenkerne 25 aus der Fig. 1 bzw. Fig. 2 fallen in die Gruppe dieses Ausführungsbeispiels. In Fig. 4 und 5 sind zwei weitere mögliche Varianten dieses Ausführungsbeispiels eines Spulenkerns 25 dargestellt.

Fig 3 c) zeigt ein weiteres Ausführungsbeispiel eines Spulenkerns 25, bei welcher die Kontaktfläche 271 an einer Fläche des Längsschenkel 26 angeordnet ist, die senkrecht auf der axialen Richtung steht. Somit ist das Polstück 27 an der Fläche am oberen zweiten Ende 262 des Längsschenkel 26 angeordnet.

Fig. 3 d) zeigt ein weiteres Ausführungsbeispiel eines Spulenkerns 25, bei welcher die Kontaktfläche 271 wiederum an einer Fläche des Längsschenkel 26 angeordnet ist, die senkrecht auf der axialen Richtung steht. Somit ist das Polstück 27 an der Fläche am oberen zweiten Ende 262 des Längsschenkel 26 angeordnet. Das Besondere an diesem Ausführungsbeispiel ist, dass das Polstück 27 gewinkelt ausgestaltet ist. Das heisst, das Polstück 27 umfasst zwei Teilstücke, von denen sich eines in radialer Richtung R und das andere in axialer Richtung A erstreckt. Das Polstück 27 weist somit die Form eines "L" auf. Die beiden Teilstücke des Polstück 27 müssen nicht zwingend die gleichen Kantenlängen besitzen. Es ist ebenso möglich, dass ein Teilstück eine längere Kantenlänge als das andere aufweist. Ebenso ist der Winkel zwischen den beiden Teilstücken nicht zwingend 90°. Es sind auch Ausführungsbeispiele möglich, in welchen einer von 90° verschiedener Winkel von den beiden Teilstücken eingeschlossen wird.

Fig. 4 zeigt eine perspektivische Darstellung einer ersten Variante des Ausführungsbeispiels eines Spulenkerns 25 aus Fig. 3 b) (oben), sowie die Draufsicht aus axialer Richtung (unten) auf diesen, also darstellungsgemäss von oben. In dieser Variante weist die Stirnfläche 272 keine Krümmung auf, sondern ist als planare Fläche ausgestaltet. Der Längsschenkel 26 ist in dieser Variante geblecht aus Elementen 263, deren Material bevorzugt Elektroblech ist, hergestellt. Das Polstück 27 ist aus einem Pulververbundwerkstoff, bevorzugt einem weichmagnetischen Pulververbundwerkstoff hergestellt.

Fig. 5 zeigt eine perspektivische Darstellung einer zweiten Variante des Ausführungsbeispiels eines Spulenkerns 25 aus Fig. 3 b) (oben), sowie die Draufsicht aus axialer Richtung (unten) auf diesen, also darstellungsgemäss von oben In dieser Variante ist die Stirnfläche 272 des Polstücks 27 als gekrümmte Fläche ausgestaltet ist. Die Rundung der Stirnfläche 272 ist koaxial zur becherförmigen Ausnehmung 211 (Fig. 11) ausgestaltet. In dieser Variante ist die Stirnfläche 272 bezüglich der Umfangrichtung nicht breiter ausgestaltet als die maximale Erstreckung der Kontaktfläche 271 in Umfangrichtung. Das heisst, die Kante 2711 schliesst mit den beiden Seitenkanten 2713 des Polstücks 27 jeweils einen 90° Winkel ein.

Selbstverständlich sind auch die beschriebenen Varianten und Ausführungsbeispiele der Spulenkerne 25 aus den Figuren 2-5 untereinander beliebig in jeglicher Form kombinierbar. Das heisst, es ist ebenso möglich, dass bei jedem der Ausführungsbeispiele eines Spulenkerns 25 aus Fig. 3 z.B. die Stirnfläche 272 des Polstücks 27 als gekrümmte Fläche ausgestaltet ist oder aber z.B. die Stirnfläche 272 bezüglich der Umfangrichtung breiter ausgestaltet ist als die maximale Erstreckung der Kontaktfläche 271 in Umfangsrichtung.

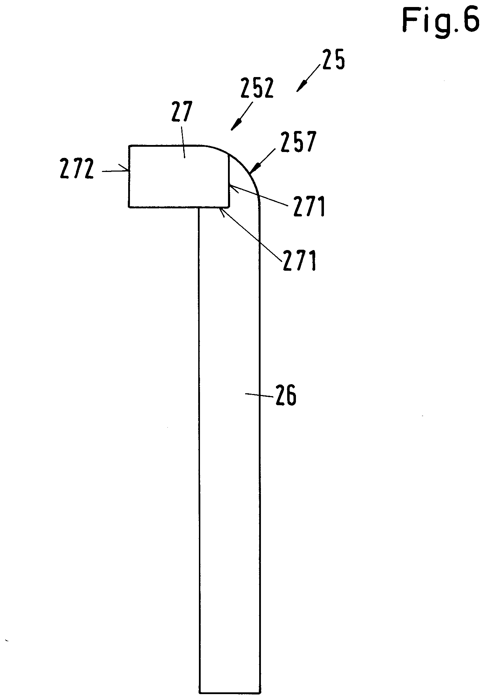

Fig. 6 zeigt eine schematische Schnittdarstellung eines weiteren Ausführungsbeispiels eines Spulenkerns 25. Hierbei weist der Spulenkern 25 an einem axial oberen Ende 252 eine Abrundung auf, welche den Spulenkern 25 aus der axialen Richtung A in die radiale Richtung R umlenkt. In diesem Ausführungsbeispiel sind der Spulenkern 25 und die Kontaktfläche 271 nach der Ausgestaltung analog der Fig. 3 b) ausgestaltet und der Spulenkern 25 weist zusätzlich eine Abrundung 257 der äusseren Kante 258 des Spulenkerns 25 am axial oberen Ende des Spulenkerns 252 auf. Das heisst in diesem Ausführungsbeispiel ist im Vergleich zu den Ausführungsbeispielen in Fig. 3 die radial aussen liegende Kante 258 am axial oberen Ende 252 des Spulenkerns 25 eine gebrochene Kante bzw. eine abgerundete Kante, wie durch die Abrundung 257 dargestellt. Je nach Ausführungsbeispiel des Spulenkerns 25 kann sich die Abrundung 257 nur in Bereichen des Längsschenkels 26 oder nur in Bereichen des Polstücks 27 oder aber sowohl in Bereichen des Längsschenkels 26 wie auch des Polstücks 27 erstrecken.

Dieses Ausführungsbeispiel hat den Vorteil, dass sie geringere Wirbelstromverluste aufweist und auch im Hinblick auf die Konstruktion einfacher umzusetzen ist. Dieses Ausführungsbeispiel kann selbstverständlich mit allen anderen Ausführungsbeispielen der Spulenkerne 25 hinsichtlich der Kontaktfläche 271, der Stirnfläche 272, der Polstücke 27 und der Längsschenkel 26 umgesetzt bzw. kombiniert werden.

Hinsichtlich des Aufbaus und der Materialien des Längsschenkels 26 und des Polstücks 27 sind die gleichen Vorzüge wie bei den in Fig. 3 erläuterten Ausführungsbeispielen eines Spulenkerns 25 von Bedeutung.

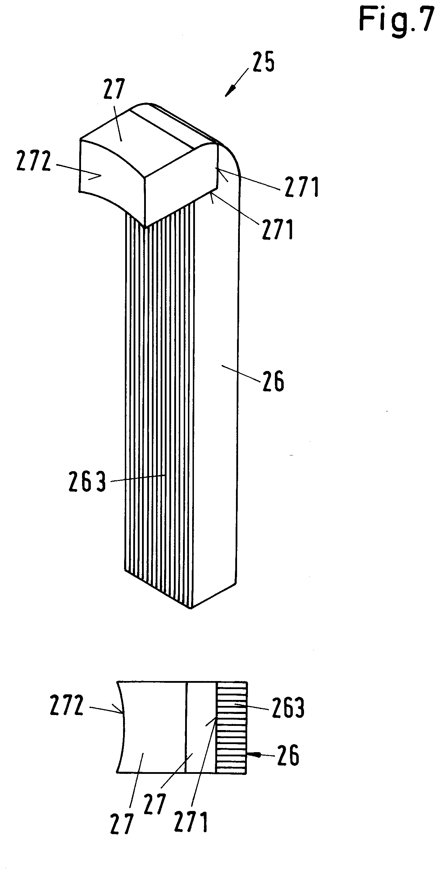

Fig. 7 zeigt eine perspektivische Darstellung einer Variante des Ausführungsbeispiels eines Spulenkerns 25 aus Fig. 6 (oben), sowie die Draufsicht aus axialer Richtung (unten) auf diesen, also darstellungsgemäss von oben. In dieser Variante ist die Stirnfläche 272 des Polstücks 27 als gekrümmte Fläche ausgestaltet, wobei die Rundung der Stirnfläche 272 koaxial zur becherförmigen Ausnehmung 211 (Fig. 11) ausgestaltet ist.

Ebenso sind auch Varianten möglich bei denen die Stirnfläche 272 des Polstücks 27 bezüglich der Umfangsrichtung breiter ausgestaltet ist als die maximale Erstreckung der Kontaktfläche 271 in Umfangsrichtung. Zudem kann die Stirnfläche 272 auch keine Rundung aufweisen, sondern einfach eine planare Fläche sein.

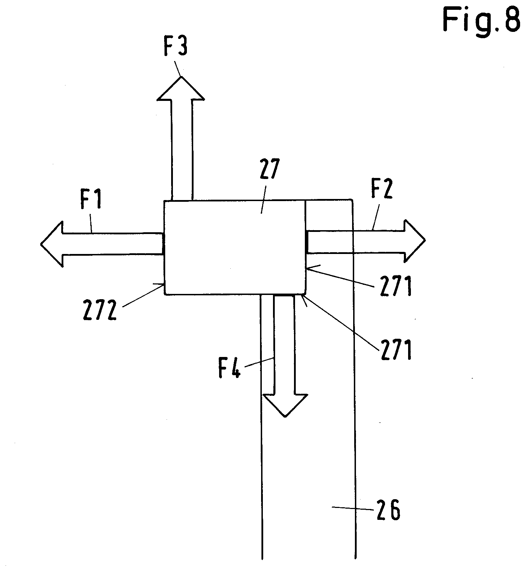

Fig. 8 zeigt eine schematische Schnittdarstellung eines Ausschnitts eines Ausführungsbeispiels eines Spulenkerns 25 zur Verdeutlichung der wirkenden Zugkräfte. Diese schematische Darstellung dient rein zur Verdeutlichung der auf das Ausführungsbeispiel des Spulenkerns 25 analog zu Fig. 3 b) wirkenden magnetischen Zugkräfte. Die vier dicken Pfeile mit den Bezugszeichen F1-F4 symbolisieren die Kraftvektoren, die an der jeweiligen Fläche wirken. Da der Rotor 3 einen magnetisch wirksamen Kern 31 aufweist, wird dieser an den Spulenkern 25, genauer an das Polstück 27 angezogen. Da es sich bei dieser Anziehung um eine Wechselwirkung handelt, muss eine entgegengesetzte gleichgrosse Kraft vorhanden sein, sodass der Rotor 3 in der Magnetlagervorrichtung 1 gelagert werden kann. Das heisst auf das Polstück 27 wirkt ebenso eine Kraft. Die auf das Polstück 27 wirkende Kraft wird über durch das Polstück 27 an den Längsschenkel 26 und somit auch an die Kontaktfläche 271 weitergegeben. Je nachdem wie die Kontaktfläche 271 ausgestaltet ist, wirken unterschiedliche Kraftvektoren auf diese. Im Fall der nicht gewinkelten Kontaktflächen 271 (vgl. Fig. 3 a) und 3 c)) wirken je nach Ausführungsbeispiel die Kräfte nur entweder in axialer Richtung A oder in radialer Richtung R. Das heisst die gesamte an der Kontaktfläche 271 wirkende Kraft wird nur durch einen Kraftvektor an den Längsschenkel 26 übertragen. Dadurch sind die Anforderungen an die Fügearten in diesen beiden Beispielen sehr hoch damit eine stabile und zuverlässige Verbindung des Polstücks 27 und des Längsschenkels 26 an der Kontaktfläche 271 hergestellt werden kann. Aus diesem Grund ist es vorteilhaft, wenn die Kontaktfläche 271 wie in Fig. 8 dargestellt derart optimiert wird, dass sie gewinkelt ausgestaltet ist. Durch dieses Ausführungsbeispiel wird die auf die Kontaktfläche 271 wirkende Kraft in zwei Kräfte F2, F4 aufgeteilt. Das heisst, es wirkt sowohl ein Kraftvektor in axialer Richtung A als auch ein Kraftvektor in radialer Richtung R auf den Längsschenkel 26. Dies hat den Vorteil, dass nur sehr geringe Belastungen durch magnetische Kräfte auf die Kontaktfläche 271 wirken und dadurch die Defektanfälligkeit der Spulenkerne 25 verringert wird.

Ein weiterer Vorteil, der sich daraus ergibt, ist, dass für eine stabile und zuverlässige Verbindung des Polstücks 27 und des Längsschenkels 26 kostengünstigere Fügearten eingesetzt werden können. Im Hinblick auf die stoffschlüssige Fügeart birgt dieses Ausführungsbeispiel einen weiteren Vorteil. Durch die gewinkelte Kontaktfläche 271 wird die verfügbare Klebefläche im Vergleich zu den nicht gewinkelten Ausgestaltungen der Kontaktfläche 271 vergrössert und somit ist eine stabilere und zuverlässigere Verbindung zwischen dem Polstück 27 und dem Längsschenkel 26 möglich.

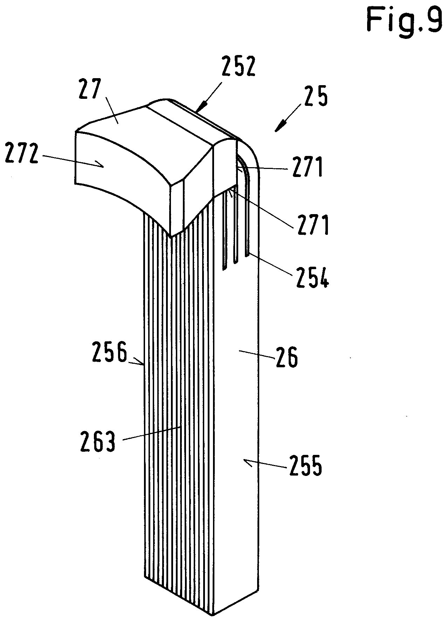

Fig. 9 zeigt eine perspektivische Darstellung eines weiteren Ausführungsbeispiels eines Spulenkerns 25. Dabei ist der Längsschenkel 26 wiederum geblecht aus Elementen 263 hergestellt, wobei die Elemente 263 in Umfangsrichtung des Stators 2 gestapelt sind, wobei jeder Spulenkern 25 eine erste laterale Begrenzungsfläche 255 und eine zweite laterale Begrenzungsfläche 256 aufweist. Das Polstück 27 weist eine Rundung der Stirnfläche 272 koaxial zur becherförmigen Ausnehmung 211 (Fig. 11) auf. Die Stirnfläche 272 ist dabei bezüglich der Umfangrichtung breiter ausgestaltet als die maximale Erstreckung der Kontaktfläche 271 in Umfangsrichtung. Das Material des Polstücks 27 ist in diesem Ausführungsbeispiel ein Pulververbundwerkstoff, bevorzugt ein weichmagnetischer Pulververbundwerkstoff. Ebenso ist es aber möglich, dass das Material des Polstücks 27 ein anderer Werkstoff sein kann. Die Kontaktfläche 271 ist L-förmig ausgestaltet und der Spulenkern 25 weist am axial oberen Ende 252 eine Abrundung auf, welche den Spulenkern 25 aus der axialen Richtung A in die radiale Richtung R umlenkt. In diesem Ausführungsbeispiel weist mindestens eine der ersten oder der zweiten lateralen Begrenzungsflächen 255, 256 drei Schlitze 254 auf. Es sind aber auch andere Ausführungen möglich, in welchen weniger als drei oder mehr als drei Schlitze 254 vorhanden sind. Die Schlitze 254 erstrecken sich nicht nur in den beiden lateralen Begrenzungsflächen 255, 256 sondern können auch in den innenliegenden Elementen 263 vorhanden sein. Es sind Ausführungen möglich, in welchen sich durch alle Elemente 263 hindurch eine Mehrzahl von Schlitzen 254 erstrecken.

Bei diesem Ausführungsbeispiel ist der Vorteil, dass die Schlitze 254 eine elektrische Isolation darstellen, wodurch sie den Pfad der Wirbelströme blockieren. Durch diese Massnahme werden die Wirbelstromverluste nochmals deutlich reduziert.

Fig. 10 zeigt eine perspektivische Darstellung eines weiteren Ausführungsbeispiels einer erfindungsgemässen Magnetlagervorrichtung 1. In der folgenden Beschreibung des weiteren Ausführungsbeispiels der Magnetlagervorrichtung 1 werden nur die Unterschiede zum ersten Ausführungsbeispiel aus Fig. 1 näher erläutert. Die Erläuterungen zum ersten Ausführungsbeispiel gelten in gleicher Weise oder sinngemäß auch für das zweite Ausführungsbeispiel. Gleiche Bezugsziffern bezeichnen die gleichen Merkmale, die unter Bezugnahme auf das erste Ausführungsbeispiel erläutert wurden, oder funktionell gleichwertige Merkmale.

In diesem Ausführungsbeispiel in Fig. 10 umfasst die Magnetlagervorrichtung 1 die in Fig. 9 dargestellten Spulenkerne 25. Ein weiterer Unterschied dieses Ausführungsbeispiels einer Magnetlagervorrichtung 1 zu dem Ausführungsbeispiel aus Fig. 1 ist, dass der Rückschluss 28 anders ausgestaltet ist. Der Rückschluss 28 ist ringförmig mit einem metallischen Band 29 ausgestaltet, welches sich von einem radial inneren Anfang 291 zu einem radial äusseren Ende 292 erstreckt. Das Band 29 formt mehrere Bandwicklungen 293, die bezüglich der radialen Richtung R flächig aneinander anliegen. Die Längsschenkel 26 werden am ersten Ende 261 durch eine axiale Stirnfläche 265 begrenzt, an welchen der Rückschluss 28 anliegt. Der Rückschluss 28 formt einen Kreisring, dessen radiale Breite gleich der radialen Breite der Stirnflächen 265 der Längsschenkel 26 ist. Das heisst, der radial innere Anfang 291 ist in axialer Richtung A bündig mit einer radial innenliegenden Innenfläche 266 des Längsschenkels 26 und das radial äussere Ende 292 ist in axialer Richtung A bündig mit einer radial aussenliegenden Aussenfläche 267 des Längschenkels 26.

Dies hat unter anderem den Vorteil, dass im Inneren des Stators 2 mehr Platz vorhanden ist, der genutzt werden kann, um andere Komponenten darin zu verbauen. Dadurch kann der Stator 2 samt Statorgehäuse 21 (Fig. 11) kleiner und kompakter gebaut werden, wodurch die Einsatzflexibilität der Magnetlagervorrichtung 1 erhöht wird.

Es versteht sich von selbst, dass alle in der Figurenbeschreibung gezeigten Ausführungsbeispiele eines Spulenkerns 25 mit ihren jeweiligen Eigenarten in jeglicher Form kombinierbar sind. Ebenso können natürlich alle Ausführungsbeispiele eines Spulenkerns 25 in den Ausführungsbeispielen einer Magnetlagervorrichtung 1 eingesetzt werden.

Des Weiteren ist es möglich, dass sämtliche gezeigten Ausführungsbeispiele eines Spulenkerns 25 derart ausgestaltet sein können, dass der dem Rotor 3 zu Verfügung stehende Platz in der Magnetlagervorrichtung 1 vergrössert wird. Dies gelingt durch eine spezielle äussere Form der Spulenkerne 25.

Dabei wird der Spulenkern 25 in einen axial unteren Abschnitt und einen axial oberen Abschnitt aufgeteilt, wobei der untere Abschnitt und der obere Abschnitt bezüglich der axialen Richtung A benachbart zueinander angeordnet sind. Das Polstück 27 ist an dem axial oberen Abschnitt angeordnet. Für jeden Spulenkern 25 weist die Stirnfläche 272 des Polstücks 27 von dem axial unteren Abschnitt des zugehörigen Längsschenkels 26 einen ersten Abstand in radialer Richtung auf, und von dem axial oberen Abschnitt einen zweiten Abstand in radialer Richtung, wobei der zweite Abstand grösser ist als der erste Abstand. Dies bedeutet, dass jeder Längsschenkel 26 derart ausgestaltet ist, dass der axial obere Abschnitt bezüglich des axial unteren Abschnitts in radialer Richtung nach aussen verschoben ist, sodass sich der für den Rotor 3 zur Verfügung stehende Platz zwischen den Stirnflächen 272 vergrössert, ohne dass dabei die Gefahr eines direkten Übertritts des magnetischen Flusses zwischen dem Längsschenkel 26 und dem magnetisch wirksamen Kern 31 des Rotors 3 besteht. Dadurch, dass die axial oberen Abschnitte bezüglich der radialen Richtung und relativ zu den axial unteren Abschnitten radial nach aussen versetzt sind, vergrössert sich der Abstand, nämlich der zweite Abstand, zwischen den Längsschenkeln 26 und den Stirnflächen 272 im Bereich der axial oberen Abschnitte. Dadurch vergrössert sich auch der Abstand zwischen dem magnetisch wirksamen Kern 31 des Rotors 3 und den Längsschenkeln 26 insbesondere im Bereich der axial oberen Abschnitte.

Solche gerade beschriebenen Spulenkerne 25 sind analog zu denen in Fig. 3 in der Europäischen Patentanmeldung EP4084304A1 abgebildeten Spulenkerne ausgebildet.

Fig. 11 zeigt eine schematische Schnittdarstellung für eine Ausgestaltung eines Statorgehäuses 21. In den Ausführungsbeispielen in Fig. 1 und Fig. 10 ist dieses Statorgehäuse 21 aus Gründen der besseren Übersicht nicht dargestellt. Fig. 11 soll lediglich der Anschauung dienen, um zu zeigen, wie eine für den Betrieb der Magnetlagervorrichtung 1 notwendige Umkapselung des Inneren des Stators 2 aussieht. Aus diesem Grund sind die anderen Komponenten des Stators 2 nur schematisch dargestellt und rein anschaulich zu verstehen.

Es sind ebenso Ausgestaltungen des Statorgehäuses 21 möglich, bei denen die becherförmige Ausnehmung 211 in eine Bohrung übergeht, die sich zentral entlang der Mittelachse des Stators 2 in axialer Richtung A durch das gesamte Statorgehäuse 21 erstreckt.

Beim Betrieb der Magnetlagervorrichtung 1 in Bereichen in denen z.B. chemisch aggressive Substanzen zum Einsatz kommen, ist es wichtig, dass das Innere des Stators 2 sicher umkapselt ist und somit vor diesen Substanzen geschützt wird. Damit trotzdem ein Rotor 3 verwendet werden kann, weist das Statorgehäuse 21 eine becherförmige Ausnehmung 211 auf, in welche der Rotor 3 eingesetzt werden kann.

## Patentansprüche

1. Magnetlagervorrichtung zur berührungslos magnetischen Lagerung eines Rotors (3), der einen scheibenförmigen oder ringförmigen magnetisch wirksamen Kern (31) aufweist, wobei die Magnetlagervorrichtung einen Stator (2) mit einer becherförmigen Ausnehmung (211) aufweist, die an einem axialen Ende des Stators (2) angeordnet ist, und in die der Rotor (3) einsetzbar ist, wobei der Stator (2) eine Mehrzahl von Spulenkernen (25) aufweist, von denen jeder einen Längsschenkel (26) und ein Polstück (27) aufweist, wobei sich jeder Längsschenkel (26) von einem ersten Ende (261) in einer axialen Richtung (A) bis zu einem zweiten Ende (262) erstreckt, wobei am zweiten Ende (262) eine Kontaktfläche (271) angeordnet ist, wobei sich jedes Polstück (27) von der Kontaktfläche (271) zumindest teilweise in einer radialer Richtung (R) bis zu einer Stirnfläche (272) erstreckt, wobei die radiale Richtung (R) senkrecht zur axialen Richtung (A) ist, wobei die Stirnfläche (272) um die becherförmige Ausnehmung (211) herum angeordnet sind, und wobei an jedem Längsschenkel (26) mindestens eine konzentrierte Wicklung (61) angeordnet ist, welche den jeweiligen Längsschenkel (26) umgibt, **dadurch gekennzeichnet, dass** die Längsschenkel (26) aus einem ersten Material gefertigt sind und die Polstücke (27) aus einem zweiten Material gefertigt sind, wobei das erste Material und das zweite Material unterschiedlich sind.

2. Magnetlagervorrichtung nach Anspruch 1, wobei jeder Längsschenkel (26) geblecht aus Elementen (263) hergestellt ist, wobei die Elemente (263) in Umfangsrichtung des Stators (2) gestapelt sind.

3. Magnetlagervorrichtung nach einem der vorangehenden Ansprüche, wobei das erste Material ein Elektroblech ist.

4. Magnetlagervorrichtung nach einem der vorangehenden Ansprüche, wobei das zweite Material ein Pulververbundwerkstoff ist, bevorzugt ein weichmagnetischer Pulververbundwerkstoff.

5. Magnetlagervorrichtung nach einem der vorangehenden Ansprüche, wobei die Kontaktfläche (271) planar ausgestaltet ist und an einer Fläche des Längsschenkels (26) angeordnet ist, die senkrecht auf der radialen Richtung steht.

6. Magnetlagervorrichtung nach einem der Ansprüche 1-4, wobei die Kontaktfläche (271) an einer Fläche des Längsschenkel (26) angeordnet ist, die senkrecht auf der axialen Richtung steht.

7. Magnetlagervorrichtung nach einem der vorangehenden Ansprüche, wobei die Kontaktfläche (271) gewinkelt ausgestaltet ist.

8. Magnetlagervorrichtung nach Anspruch 7, wobei die Kontaktfläche (271) zwei Teilflächen aufweist, wobei die erste Teilfläche (273) senkrecht auf der axialen Richtung steht und die zweite Teilfläche (274) senkrecht auf der radialen Richtung steht.

9. Magnetlagervorrichtung einem der vorangehenden Ansprüche, wobei das Polstück (27) gewinkelt ausgestaltet ist, wobei das Polstück (27) zwei Teilstücke umfasst, von denen sich eines in radialer Richtung erstreckt und das andere in axialer Richtung.

10. Magnetlagervorrichtung nach einem der vorangehenden Ansprüche, wobei der Spulenkern (25) an einem axial oberen Ende (252) eine Abrundung aufweist, welche den Spulenkern (25) aus der axialen Richtung (A) in die radiale Richtung (R) umlenkt.

11. Magnetlagervorrichtung nach einem der vorangehenden Ansprüche, wobei die Stirnfläche (272) des Polstücks (27) als gekrümmte Fläche ausgestaltet ist.

12. Magnetlagervorrichtung nach Anspruch 10, wobei die Rundung der Stirnfläche (272) koaxial zur becherförmigen Ausnehmung (211) ausgestaltet ist.

13. Magnetlagervorrichtung nach einem der vorangehenden Ansprüche, wobei die Stirnfläche (272) bezüglich der Umfangrichtung breiter ausgestaltet ist als die maximale Erstreckung der Kontaktfläche (271) in Umfangsrichtung.

14. Magnetlagervorrichtung nach einem der vorangehenden Ansprüche, wobei der Stator (2) zur Erzeugung eines Drehmoments ausgestaltet ist, mit welchem der Rotor (3) berührungslos magnetisch zur Rotation um die axiale Richtung (A) antreibbar ist.

15. Elektromagnetischer Drehantrieb, der als Tempelmotor ausgestaltet ist, **dadurch gekennzeichnet, dass** der elektromagnetische Drehantrieb eine Magnetlagervorrichtung (1) gemäss Anspruch 14 umfasst, sowie einen Rotor (3) mit einem scheibenförmigen oder ringförmigen magnetisch wirksamen Kern (31), wobei der Rotor (3) in die becherförmige Ausnehmung (211) einsetzbar ist, und wobei der Rotor (3) als Rotor (3) des elektromagnetischen Drehantriebs ausgestaltet ist.
